# EUROPEAN PATENT SPECIFICATION

(11) **EP 3 223 711 B1**
(45) Date of publication and mention of the grant of the patent: **15.09.2021**
(21) Application number: 15801280.7
(22) Date of filing: 24.11.2015
(51) Int. Cl.: A61B 8/00

(54) **A MULTI-SENSOR ULTRASOUND PROBE**
MULTISENSOR-ULTRASCHALLSONDE
SONDE À ULTRASONS À MULTIPLES CAPTEURS

(30) Priority: 25.11.2014 US 201462084147 P
(43) Date of publication of application: 04.10.2017
(73) Proprietor: Koninklijke Philips N.V., 5656 AG Eindhoven (NL)
(72) Inventor: VAN ALPHEN, Daniel, 5656 AE Eindhoven (NL); POLAND, McKee Dunn, 5656 AE Eindhoven (NL); HOPE SIMPSON, David, 5656 AE Eindhoven (NL); CANFIELD, Earl M., 5656 AE Eindhoven (NL); MESAROS, Robert, 5656 AE Eindhoven (NL); FREEMAN, Steven Russell, 5656 AE Eindhoven (NL)
(74) Representative: Philips Intellectual Property & Standards
(86) International application number: PCT/IB2015/059058
(87) International publication number: WO 2016/083985

(56) References cited:
- JP-A- S63 290 549
- US-A1- 2006 058 655
- US-A1- 2009 312 643
- US-A1- 2010 298 713
- US-A1- 2012 108 975
- US-A1- 2013 172 757
- US-B1- 6 261 234

## Description

This invention relates to ultrasonic diagnostic imaging systems and methods, such as the use of an ultrasound probe having several arrays configured for multi-faceted ultrasound exams.

The journey from home to hospital is critical for every patient faced with a medical emergency, because the sooner you can connect a patient to quality care, the better the outcome will be. A key aspect of this care is streamlining communication among caregivers every step of the way. Then caregivers can have a more complete picture of a patient's medical condition and can make more informed decisions across the entire continuum from home to hospital and everywhere in between.

US 2010/298713 A1 discloses an ultrasonic transducer assembly for diagnostic imaging which includes a housing, a plurality of image data acquisition transducer arrays, a transducer controller assembly, selection means for indicating a selected one of the plurality of ultrasonic image data acquisition transducer arrays to the transducer controller assembly, and a communication assembly for sending ultrasound image data and for receiving transmit waveforms and/or control data.

Further ultrasonic transducer assemblies with more than one transducer arrays are known from US 2012/108975 A1, US 2013/172757A1, and US 2009/312643 A1.

Ultrasound images are an important part of this continuum. There is a need to bring ultrasound technology to the patient wherever care takes place and unfortunately, limited options are currently available.

The present invention as claimed in claim 1 includes a wireless ultrasound probe. The probe includes a probe housing having a plurality of sides, such as a first side and a second side, as well as a grip portion. The first side includes a first transducer array and the second side includes a second transducer array. The first transducer array is a high frequency transducer array and the second transducer array is a low frequency transducer array. The high frequency and low frequency transducer arrays are configured to operate in different scanning modes. The probe includes at least one micro-beamformer coupled to the first and second transducer arrays, wherein the at least one micro-beamformer includes a plurality of micro-beamformer channels which are configured to operate the first and second transducer arrays simultaneously, wherein each channel comprises a first transmitter for driving the first transducer array and a second transmitter for driving the second transducer array. The probe further includes a processor in the housing configured to select between the first or second transducer array for an ultrasound scanning procedure, and an image processor configured to generate ultrasound image data for display to a user.

In the drawings:
FIGURE 1 illustrates an ultrasound probe according to a first embodiment of the present invention.
FIGURES 2A and 2B illustrate ultrasound probes according to a second embodiment of the present invention.
FIGURES 3A-3C illustrate example probe configurations for use of different arrays in an ultrasound probe.
FIGURE 4 illustrates in block diagram form example electronic subsystems between a microbeamformer and antenna of a wireless probe of the present invention.
FIGURE 5 illustrates an ultrasound probe having different arrays for different applications, in accordance with the principles of the present invention.
FIGURE 6 illustrates in block diagram form a channel of a microbeamformer ASIC configured to transmit and receive ultrasound at multiple frequencies.
FIGURE 7 illustrates in block diagram form the receive configuration of the microbeamformer ASIC of FIGURE 6.
FIGURE 8 illustrates an example embodiment of an ultrasound probe with high and low frequency transducers.
FIGURES 9A and 9B illustrate a single transducer array with both high and low frequency transducer elements suitable for operation with a multifrequency microbeamformer ASIC of the present disclosure.
FIGURE 10 illustrates a workflow using an ultrasound probe, in accordance with the principles of the present invention.
FIGURE 11 illustrates communication of data generated from an ultrasound probe, according to an embodiment of the present invention.

In accordance with the principles of the present invention, an ultrasonic diagnostic imaging system and probe are described in which an ultrasound probe can be used for multi-faceted exams, such as for triage and emergency. The probe can include different transducer arrays, such as a linear, a curved linear, and a sector array that are combined into a single handheld unit that can be, for example, coupled to a wireless display for displaying ultrasound images. Related methods are provided, such as a method for automatically selecting the appropriate array for the user to scan with based on the intended exam and/or location on the body of a patient.

In one aspect, the present invention includes ultrasound probes. For example, the present invention includes a wireless ultrasound probe that can include a probe housing having several sides (e.g., two, three, or four sides). The probe can further include a grip portion. Sides of the probe can include transducer arrays that can be arranged such that when a patient is being scanned with one array, the other array(s) are arranged as part of the hand grip for the sonographer. For example, one transducer array (e.g., a linear array) can be arranged with respect to the probe housing such that it is enclosed by a grip of the user's hand when scanning with a different transducer array (e.g., a curved linear array). The probes can further include transmit and receive circuitry and/or at least one beamformer coupled to the arrays to operate the arrays in different scanning modes. The arrays can be one-dimensional or two-dimensional arrays, and can include linear arrays, curved linear arrays, and/or sector or phased arrays. The probes can include circuitry and other electronics, such as processors, to select between the different arrays. Image processing can also be carried out in the probe, and then transmitted wirelessly to a display communicating with the probe.

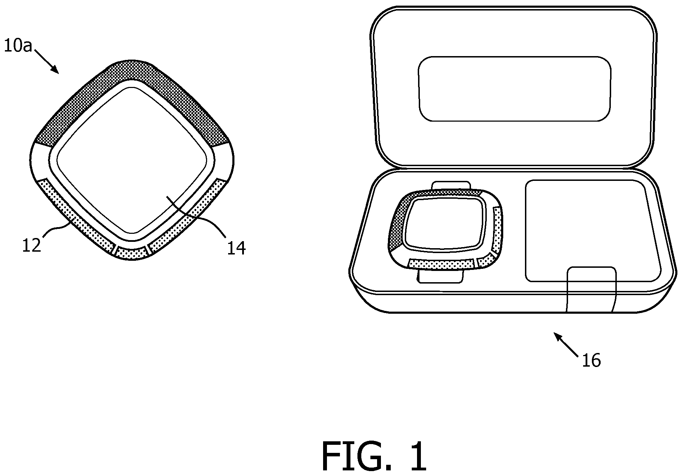

Referring to FIGURE 1, an ultrasound probe 10a of the present invention is designed to be handheld and can include four sides. A variety of shapes can be used. For example, the probe 10a can be in the shape of a square, a rectangle, a triangle, or a trapezoid. Edges, as shown, can be beveled and shaped smoothly so as to allow comfortable grip from several sides. A central indentation, possibly with anti-slip ridges, can facilitate a solid grip even when the probe is slippery. On several sides of the probe, different sensors or arrays 12 are mounted or incorporated into the probe housing, allowing the sensor in use to be facing downward while the unused sensors are enclosed by the grip of the user's hand. An indicator component 14, such as integrated light band, can be located next to each sensor. One light band can be illuminated to clearly show which sensor is active, and therefore which orientation to hold the probe for scanning. The probe can also be also wireless, so there is no need for an emanating cable to get in the way of the ergonomics or complicate the positioning. Some embodiments include the option to couple a cable (e.g., a USB cable) to the probe, which can be configured to transmit images and/or control signals to an external display or other system components, such as a mainframe ultrasound system. Another attribute of the probe is that the sensors which are not used are nestled in the hand, effectively becoming part of a comfortable grip, owing to the fact that the part pressing against the palm is a smooth lens or bezel surface on the probe. As also shown in FIGURE 1, the probe can be stored in a storage device 16. The storage device 16 can include, e.g., spare batteries and/or inductive charging devices to charge the probe when not in use.

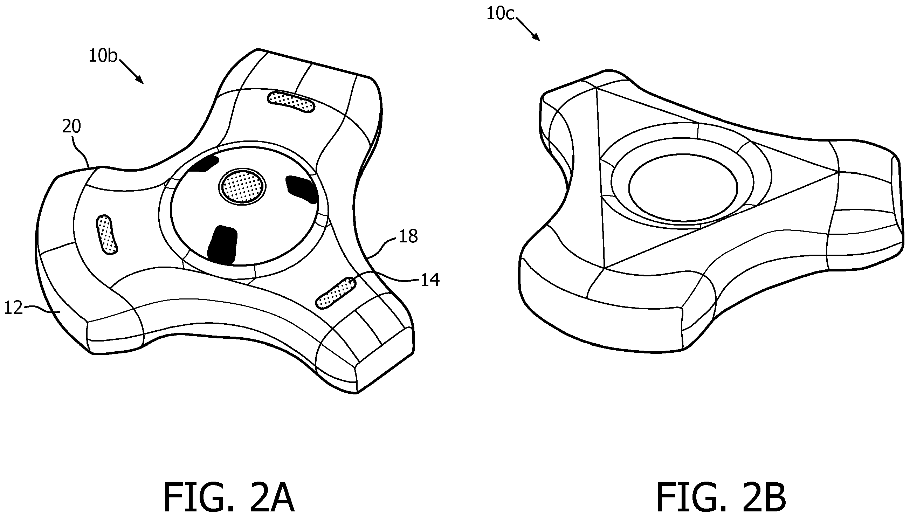

FIGURES 2A and 2B show another embodiment for probes of the present invention. As shown in FIGURES 2A and 2B, the probe 10b (top-side view) and 10c (bottom-side view) can include three array transducers, such as a curved array transducer, a linear array transducer, and a sector array transducer. Each array can be configured for different clinical applications. With the embodiments in FIGURES 2A and 2B, indented portions of the probe housing can be used as a grip portion 18 for the hand of a user opposite to the array that is used for scanning during a procedure. Alternatively, a user can wrap their hand around the extended portion 20 of the housing that extends outward towards the array transducer. In addition, indicator components 14 can be used to identify which array is ready or being used for scanning. An additional protruding structure on the probe housing can be used add additional gripping capability, battery or power storage, and/or additional indicator components or buttons for control of the probe. As shown in FIGURE 2B, an indented portion can be included in the probe housing to house, e.g., an inductive charging coil configured to charge the probe.

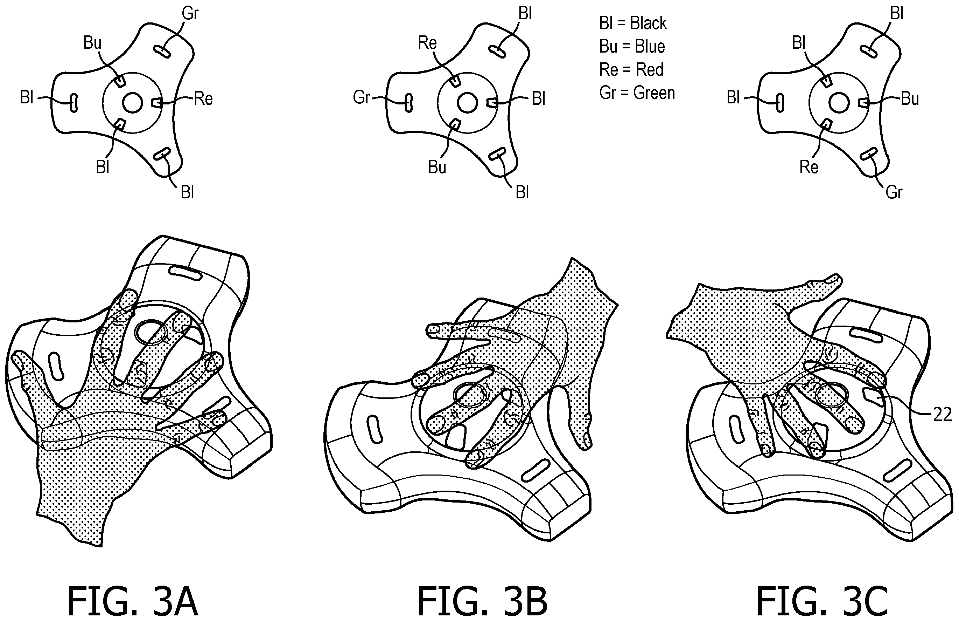

As described herein, the probes of the present invention can include an indicator component 14 to highlight which of the arrays is being operated during a scanning procedure. The present invention can further include an activation component 22, such as a button, on the probes to allow for a consistent user interface for each transducer array on the probe. For example, as shown in FIGURES 3A-C, the multisensor probe can include various activation components (e.g., buttons) located on the top of the probe and capable of changing color, lighting up, or providing an indication during operation of the probe. In FIGURE 3A, the user can hold the probe such that the linear array is in operation and two buttons on either side of the top of the probe can be used to control a feature of the ultrasound system. For example, one button can be configured to initiate a "Freeze" operation and the other button can be configured to initiate an "Acquire" operation. To provide a consistent user interface for each array, the buttons can be configured to be operable in conjunction with a particular array being used during scanning. As shown in FIGURE 3B, the curved array is operating and the activation components corresponding to the curved array are operable. Similarly, in FIGURE 3C the sector array is being operated, and the corresponding buttons are enabled to control features on the system, such as Freeze and Acquire. As shown for all three embodiments, one indicator component 14 lights up as green, for example, to show which array is being used. The other two indicator components associated with the other arrays can be black, or not lit, thereby showing the array is not in use. Furthermore, activation components 22 can be selectively colored, e.g., in red, blue, and black, such that red and blue indicate that the activation components can be activated to control some aspect of the user interface, such as, e.g., "Freeze" or "Acquire."

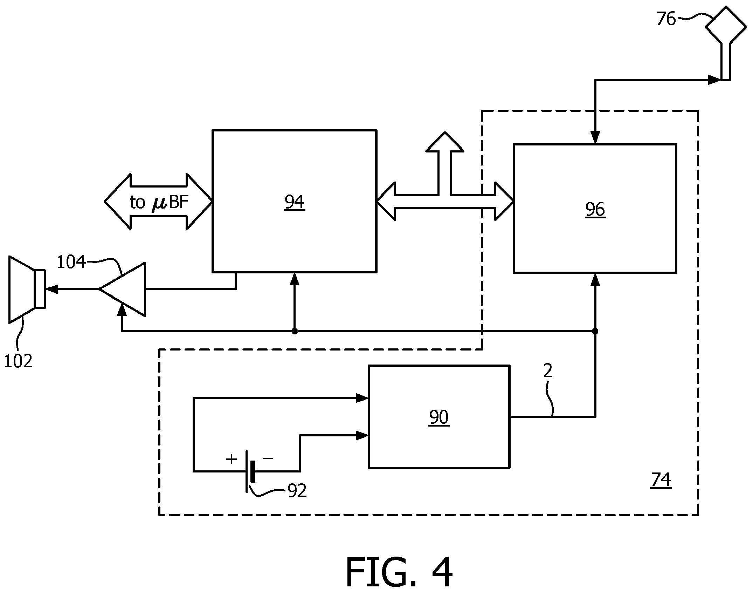

An example probe controller and transceiver subsystem for a wireless probe of the present invention is shown in FIGURE 4. A battery 92 powers the wireless probe and is coupled to a power supply and conditioning circuit 90. The power supply and conditioning circuit translates the battery voltage into a number of voltages required by the components of the wireless probe including the transducer array. A typical constructed probe may require nine different voltages, for example. The power supply and conditioning circuit also provides charge control during the recharging of the battery 92. In a constructed embodiment the battery is a lithium polymer battery which is prismatic and can be formed in a suitable shape for the available battery space inside the probe case.

An acquisition module 94 provides communication between the microbeamformer and the transceiver. The acquisition module provides timing and control signals to the microbeamformer, directing the transmission of ultrasound waves and receiving at least partially beamformed echo signals from the microbeamformer, which are demodulated and detected (and optionally scan converted) and communicated to the transceiver 96 for transmission to the base station host. A suitable acquisition module can be found, e.g., in WO2008/146208. In this example the acquisition module communicates with the transceiver over a parallel or a USB bus so that a USB cable can be used when desired, as described below. If a USB or other bus is employed, it can provide an alternative wired connection to the base station host over a cable, thus bypassing the transceiver portion 96 as described below.

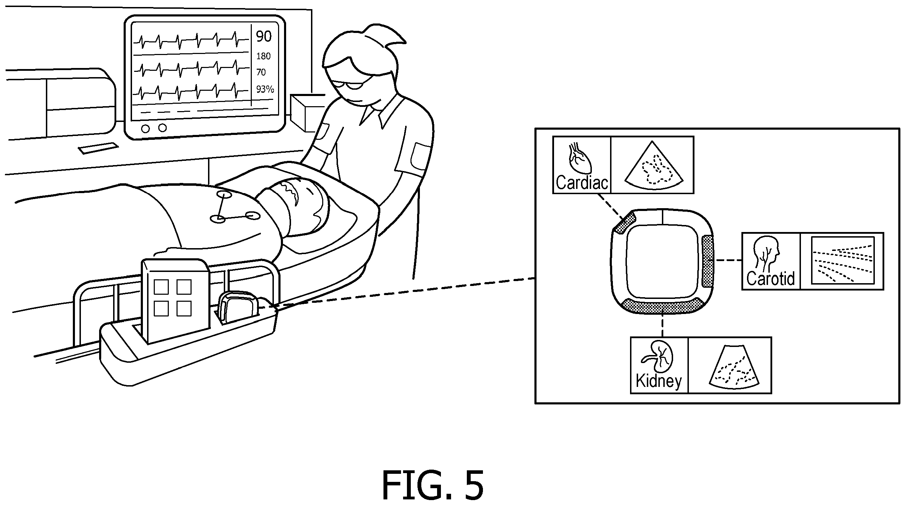

FIGURE 5 shows an example embodiment for using an ultrasound probe of the present invention. As shown, the wireless ultrasound probe can be readily stored in a variety of ways, such as near the bedside of a patient and in wireless communication with at least one display and/or tablet device. Here, the ultrasound probe is configured to include different transducer arrays, such as three transducer arrays, that operate for different clinical scanning applications, e.g., for scanning different tissues and/or organs in a patient. For example, a sector array on a side of the probe can be used for cardiac imaging, a linear array on another side of the probe can be used for carotid imaging, and a curved linear array on another side of the probe can be used for renal imaging. It is noted that all of the necessary circuitry and electronics for transmit and receive functionality, as well as image processing can be included within the transducer probe such that image data transmitted wirelessly can be simply displayed on a remote display. Furthermore, a single ultrasound acquisition signal path can be arranged in the housing and coupled to the different arrays in the probe to conserve space and provide energy efficiency. One or more microbeamformers can be included in the probe and configured to selectively beamform signals generated from the different transducer arrays.

Microbeamformers are known and described, e.g., in WO 007099473.

As described further herein, the ultrasound probes of the present invention can include a microbeamformer ASIC (application-specific integrated circuit) configured to transmit ultrasound from different arrays. For example, the microbeamformer can be used to operate two to three different arrays, such as those described in FIGURES 1, 2 and 3. In some embodiments, the different arrays can be operated at different frequencies, e.g., at both high and low frequencies.

It is noted that high and low frequency are generally described in relation to each other, so a high frequency array will transmit a higher center frequency than a low frequency array that transmits a lower center frequency. Arrays of transducer elements are configured to transmit and receive ultrasound over a bandwidth associated with a particular center frequency. For example, "high frequency" can range from 3-7 MHz (80% bandwidth at a 5MHz center frequency). "Low frequency" can range from 2-4.5 MHz (78% bandwidth at 3.2 MHz center frequency. Other ranges are available, but the two frequency ranges can overlap so that echoes of interest can be received by arrays with different frequency characteristics. Similarly, "high voltage" refers to voltages in the tens of volts, such as voltages greater than +30V or less than -30V. In some instances, high voltage devices are +35V or - 35V supplies. "Low voltage" refers to voltages in the single digits, such as 1.5V to 5V. In some instances, the low voltages are 3.3V or 5V.

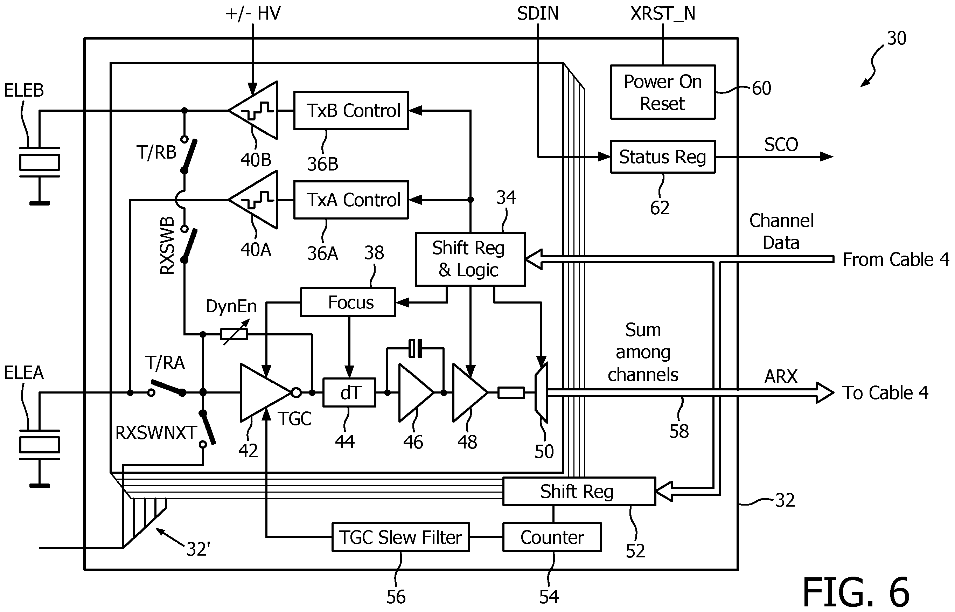

A microbeamformer ASIC 30 of the present disclosure is shown in block diagram form in FIGURE 6. The microbeamformer is constructed as a plurality of channels 32, one of which is shown in the drawing. Other identical channels are represented at 32'. Each channel can control one or more elements of a transducer array. In the implementation of FIGURE 6, the illustrated channel 32 is shown controlling two transducer elements, ELEA and ELEB. A shift register and logic circuit 34 receives channel data from the main system, which instructs the channel how to transmit ultrasound and process received ultrasound signals for the image desired by the clinician. The channel data controls two transmit control circuits 36A and 36B, which determine the nature of the transmitted pulse or waveform, e.g., its frequency, and the times at which each transmit control circuit transmits a pulse or waveform. The appropriate waveforms are amplified by high voltage transmitters 40A and 40B and the high voltage transmit signals are applied to the transducer elements ELEA and ELEB. A portion of the channel data is used to control receive circuitry of the channel including a focus control circuit 38. The focus control circuit 38 enables a TGC amplifier 42 to begin amplifying received echo signals from one or more of the transducer elements coupled to the channel. The focus control circuit also sets the delay to be applied to received echo signals by a delay circuit 44 for proper focusing of the received signals in combination with echo signals received by other channels of the microbeamformer 30. The gain applied by the TGC amplifier as echoes are received from increasing depths along the beam is controlled by TGC circuitry. A portion of the channel data is loaded into a shift register 52 which is used by a counter 54 to condition a TGC slew filter 56. The resultant TGC signal is used to dynamically control the gain of the TGC amplifier as echoes are received from a transducer element. The TGC circuitry thus applies a time gain control signal in accordance with the TGC characteristic chosen by the clinician.

The amplified and delayed receive signals are buffered by the amplifier 46 for application to a cable driver 48 which generates a voltage to drive a conductor of the cable 4. A multiplexer 50 directs the channel output signals to an appropriate microbeamformer output line 58 where they are summed together with the receive signals of other channels as necessary for beamforming. A sum signal ARX is coupled to the main system over a conductor of the cable 4.

The microbeamformer can include a power on reset circuit 60 which resets the microbeamformer to an initial state when power is first applied to the microbeamformer. A status register 62 accumulates status data from the channels which is returned to the system as SCO data to inform the main ultrasound system as to the operational status of the microbeamformer 30.

The microbeamformer channel 32 has two transmit/receive (T/R) switches T/RA and T/RB which are used to protect the input of the TGC amplifier 42 by opening the connections between the transducer elements and the TGC amplifier when the transmitters 36A and 36B are applying high voltage transmit signals to the transducer elements. The T/R switches also serve to select the receive signals from the two elements for receive processing. When T/RA is closed, receive signals from ELEA are coupled to the TGC amplifier 42. When T/RB and RXSWB are closed, receive signals from ELEB are coupled to the TGC amplifier. When all three of these switches are closed, signals received by both transducer elements are coupled to the TGC amplifier. A fourth switch, RXSWNXT, is closed to couple signals received by ELEA and/or ELEB to the receive circuitry of other channels, where they may be processed in combination with signals received from other transducer elements. This RXSWNXT switch also enables signals received on other channels to be coupled to the input of TGC amplifier 42 for summation and concurrent processing with signals received by elements ELEA and/or ELEB on that channel.

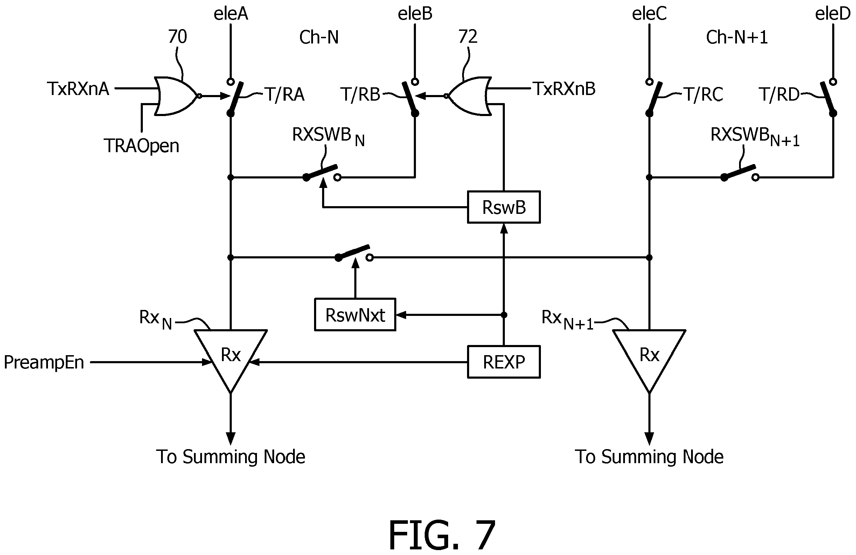

FIGURE 7 shows the receive signal circuitry of two microbeamformer channels to illustrate how the signals received by more than two transducer elements may be combined and processed by the microbeamformer. The left channel Ch-N is coupled to two transducer elements, eleA and eleB. The T/R switches T/RA and T/RB are controlled by logic gates 70 and 72 to open when the high voltage transmitters (not shown) pulse the elements to transmit ultrasound, and close the T/R switches after transmission when echo signals are to be received. Either one or both of the T/R switches may be closed to select one or both of the elements for reception. For instance, when echoes are to be received only from element eleA, only the T/RA switch is closed during reception. When echoes are to be received only from element eleB, the T/RB switch and the RXSWB_{N} switch (under control of RswB logic) are closed and switch T/RA is left open. Reception can commence with only one element, with the second element coupled in during reception for dynamic expansion of the receive aperture as echoes are received from deeper in the body. For reception by both elements, all three switches are closed to couple received signals to preamplifier Rx (e.g., the TCG amplifier 42) which is enabled for receive signal processing by an enable signal PreampEn. The time at which the preamplifier Rx of the channel begins to process received signals is controlled by the REXP logic.

A switch RXSWNXT is controlled by RswNxt logic when it is desired to combine echo signals from eleA and/or eleB with echo signals from other channels, or to process their signals through preamplifiers of other channels. A continuing series of RXSWNXT switches between channels enables echo signals of eleA and/or eleB to be directed to any other channel of the microbeamformer. The illustrated RXSWNXT switch can be closed to couple echo signals from eleA and/or eleB to the preamplifier of the second channel shown, Ch-N+1, for processing by its preamplifier RxN+1 alone or in combination with echo signals from elements eleC and eleD. So for instance, reception can begin with echo signals from element eleA with echo signals from eleB coupled in later, followed by the later addition of echo signals from element eleC and then element eleD with the closure of switch RXSWBN followed by switch RXSWB_{N+1+}. The initial stage of the high voltage T/R(A-D) switches would have been closed at the beginning of receive. Depending on the relative orientation of the elements in the array, this operation can facilitate dynamically expanding apertures in the azimuth direction, in elevation, or both. With no delay applied in this process, it can be useful for expanding aperture in elevation where the array has a lens to generate a receive focus in the field of view. The outputs of the preamplifiers are coupled to summing nodes for combining after time delay with other signals from other channels, such as the illustrated summing node line 58 in FIGURE 6.

The present disclosure describes a beamforming architecture that can be used to operate two or more different arrays operating at different frequencies. In some embodiments, a first array of transducer elements can be positioned on the same end of a probe as a second array, and the first array can operate at a different or same frequency, e.g., at higher frequencies while the second array operates at lower frequencies. In certain embodiments, the first array can be positioned on an opposing side of the probe as the second array which points sound in a different direction than the first array. In some embodiments, three or more arrays can be positioned at different locations with respect to each other on a probe enclosure, as shown e.g., in FIGURES 1-3. Each of the arrays in such an instance can be configured to operate at the same or different frequencies. For example, the first array can operate a lower frequency than the second and third array, and the second array can operate at a lower frequency than the third array. This flexibility in arranging the arrays in different positions and with different frequencies is enabled by the microbeamformer ASIC described herein.

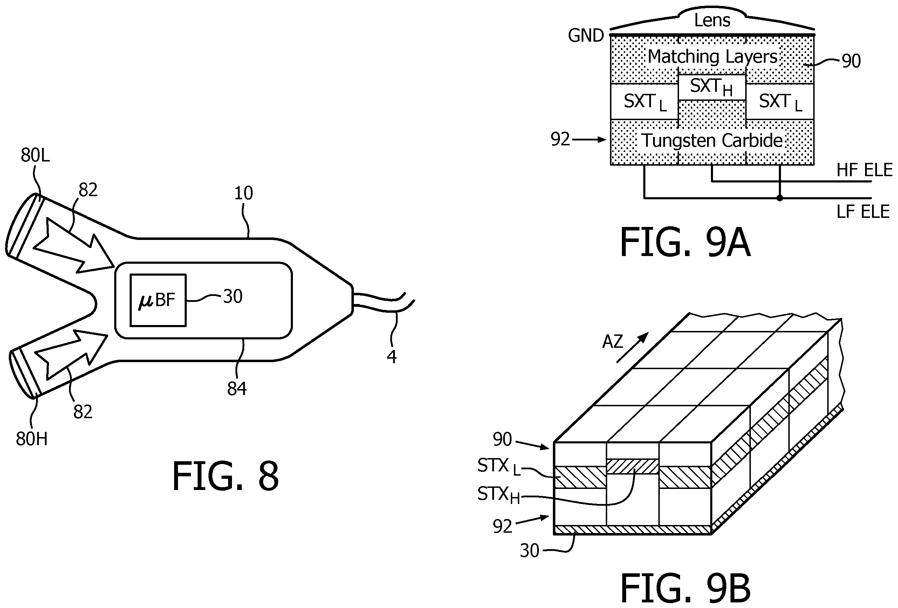

FIGURE 8 illustrates a dual array probe 10 which can be implemented using the microbeamformer of FIGURES 6 and 7. The probe 10 has two distal ends, one mounting a low frequency array transducer 80L and the other mounting a high frequency array transducer 80H. The arrays are coupled to a microbeamformer ASIC 30 located on a printed circuit board 84 in the handle of the probe. Each array is coupled to the microbeamformer by an interposer 82 which is coupled to the elements of an array at one end and to the ASIC 30 at the other end. Interposers are well known in the art as described in US patent publication no. 2008/0229835 (Davidsen et al.) and US Pat. 8,330,332 (Weekamp et al.) In some implementations, flex circuits can also be used to connect the transducer elements to the microbeamformer ASIC. The clinician can press one distal end of the probe against the skin of the patient and perform low frequency imaging, and can simply reposition the probe to press the other distal end against the patient to perform high frequency imaging, all without the need to change probes. The microbeamformer channels can operate both arrays simultaneously, using one transmitter of each channel to drive the high frequency array 80H and the other transmitter of each channel to drive the low frequency array 80L. Images from both arrays can be displayed on a display, or only images from a selected transducer can be displayed. The array for imaging can be selected by a user, for example, or in some embodiments, an image processor in the probe can be used to automatically identify which array is active by determining which array is generating an image due to being positioned on a patient for scanning. An alternative probe configuration is to locate the high and low frequency arrays side-by-side in the distal end 6 of a probe with one distal end such as probe 10 shown in FIGURE 8.

Another probe implementation not in accordance with the present disclosure is shown in FIGURES 9A and 9B, in which a central high frequency array of elements SXT_{H} is driven by one transmitter of a microbeamformer channel 32, and the other transmitter of the channel drives two elevationally positioned rows of low frequency elements SXT_{L} which are located on either side of the central array. FIGURE 9A is a crosssectional view through an elevational plane of the transducer arrays showing low frequency elements SXT_{L} located on either side of a high frequency element SXT_{H}. The rows of elements extend in the azimuth direction as shown in FIGURE 9B. The high and low frequency elements are of different shapes, sizes, and/or aspect ratios as shown in the drawings, with the high frequency elements SXT_{H} being thinner than the low frequency elements SXT_{L} in this example. On top of the elements are matching layers 90 which match the transducer element impedances to the impedance of a human body. A ground plane GND overlays the matching layers which are electrically conductive to ground the top electrodes of the transducer elements for patient safety. An acoustic lens of a polymeric material overlays the ground plane. The bottom electrode of the high frequency transducer elements is coupled a high frequency transmitter of a microbeamformer channel by a conductor HF ELE, and the bottom electrodes of the low frequency transducer elements are to the low frequency transmitter of the channel by a conductor LF ELE. This connection is made with tungsten carbide interposers 92 which, together with the matching layer thicknesses, equalize the height of the transducer stack for the different size transducer elements.

The transducer elements from different arrays can be coupled to an ASIC in a variety of ways. In some embodiments, the transducer elements can be coupled to a flex circuit behind the array (e.g., conductors HF ELE and LF ELE), which is coupled to a connector and a PCB housing an ASIC. In some embodiments, the transducer elements can be mounted on the microbeamformer ASIC 30 as shown in FIGURE 9B. In some instances, flip-chip technology can be used in mounting the array to the microbeamformer. The electrical connections, which also bond the microbeamformer ASIC to the transducer stack, may be formed by solder bump bonding or with conductive epoxy to bond pads of the ASIC.

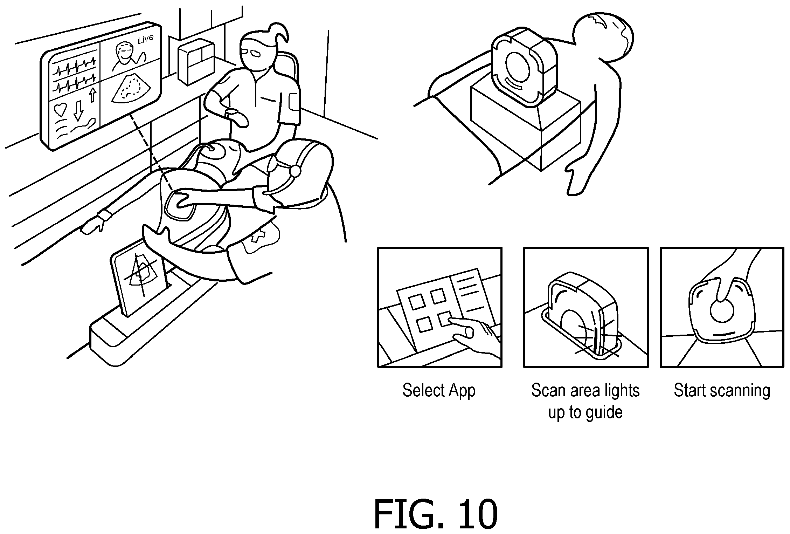

In some embodiments, the present invention includes an ultrasound system that includes a display and an ultrasound probe described herein. The display and/or the probe can be configured to allow selection of different imaging presets for different scanning applications. In some embodiments, the scanning mode presets can be selected according to a user input. For example, as shown in FIGURE 10, a clinician can select an imaging preset on a tablet display. The user input can include, e.g., a button or touch-based selection device on the probe or the display. The tablet in FIGURE 10, e.g., shows three imaging presets that correspond to the different arrays in the ultrasound probe. In certain aspects, the "preset" can be shown as a location on a graphically depicted body. Based on the selection of the imaging preset, the system automatically chooses the sensor on the probe accordingly, and highlights it with the corresponding illuminated light band. The user scans with the sensor that is lit up. During scanning, the images can be easily communicated to the associated tablet, a larger separate display, or both.

In some embodiments, ultrasound probes of the present invention can include a motion sensor that adds more intelligent automation. In certain embodiments, an ultrasound system in combination with an ultrasound probe having a motion sensor can automatically modify system operating conditions based on position information of the probe. For example, the system can automatically select a scanning mode preset from a plurality of presets based on the position information. In some aspects, the system can automatically display body markers, anatomical atlas images, labels, or other clinical information based upon the position information. For example, the system can display a representation of the patient's body showing the approximate locations of the acquired images together with selectable links to those images and/or associated data. In certain aspects, the motion sensor can be used to automatically detect the location on the body for scanning by first touching natural body landmarks, i.e. both shoulders and both hips, thereby providing a position calibration for a particular patient. Using these fiducials, the ultrasound system detects what part of the body is being scanned after subsequent repositioning of the probe, then selects the corresponding preset for the probe, and then selects the corresponding array to be used for scanning. For a health care worker untrained in ultrasound, the system provides necessary guidance and automation. For any sonographer, it provides convenient workflow.

The position information can be used for other purposes. For example, the system uses the position information to aid in the volume rendering and/or analysis of the acquired ultrasound data, e.g., from freehand sweeps and panoramic imaging.

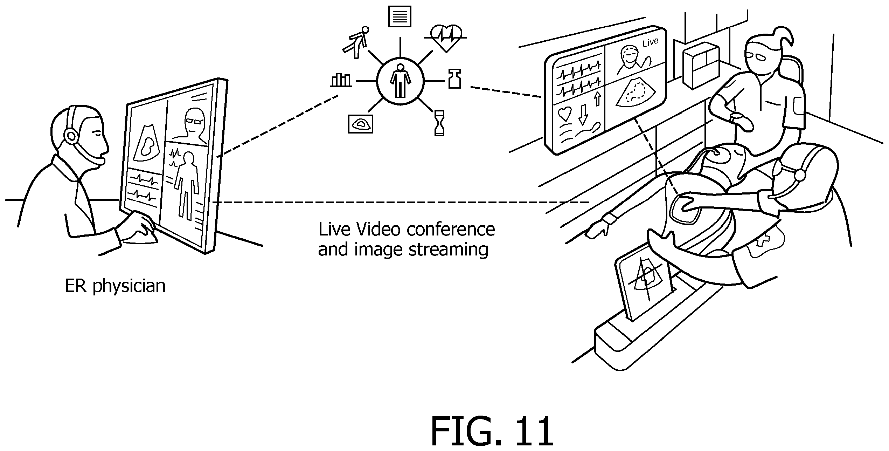

In some aspects, the probes of the present invention can be readily connected to the internet or other network to send data to other clinicians for review of the images. As shown in FIGURE 11, an EMT performing a scan in the ambulance during transport can use the ultrasound probe to search for the presence of free fluid in a patient. If such a finding is confirmed by a remotely located ER doctor, then the ER doctor can quickly decide to order an ultrasound FAST exam on patient arrival. Moreover, the ultrasound systems can include intelligent algorithms that guide the user through a protocol specific for a particular trauma or accident suffered by the patient, e.g., such as a fall down the stairs which would include looking for areas of fluid that would indicate the patient has internal bleeding.

One skilled in the art will immediately recognize that an ultrasound system in accordance with the present invention can be constructed using hardware, software, or a combination of both. In a hardware configuration the system can contain circuitry performing the described invention, or used advanced digital circuitry such as an FPGA with gates configured to perform the claimed processing. Moreover, it will be understood that various aspects, such as the processor and image processor, of the systems and methods disclosed herein, can be implemented by and/or programmed by computer program instructions. These program instructions may be provided to a processor to produce a machine, such that the instructions, which execute on the processor, create means for implementing the actions specified in the block diagram block or blocks or described for the systems and methods disclosed herein. The computer program instructions may be executed by a processor to cause a series of operational steps to be performed by the processor to produce a computer implemented process. The computer program instructions may also cause at least some of the operational steps to be performed in parallel. Moreover, some of the steps may also be performed across more than one processor, such as might arise in a multi-processor computer system.

The computer program instructions can be stored on any suitable computer-readable hardware medium including, but not limited to, RAM, ROM, EEPROM, flash memory or other memory technology, CD-ROM, digital versatile disks (DVD) or other optical storage, magnetic cassettes, magnetic tape, magnetic disk storage or other magnetic storage devices, or any other medium which can be used to store the desired information and which can be accessed by a computing device.

## Claims

1. A wireless ultrasound probe (10), comprising:
a probe housing having a first side, a second side, and a grip portion, wherein the first side comprises a high frequency transducer array (80H) and the second side comprises a low frequency transducer array (80L), wherein the high frequency and low frequency transducer arrays (80H, 80L) are configured to operate in different scanning modes;
at least one microbeamformer (30) coupled to the high frequency and low frequency transducer arrays (80H, 80L);
a processor in the housing configured to select between the high frequency or low frequency transducer array (80H, 80L) for an ultrasound scanning procedure; and
an image processor configured to generate ultrasound image data for display to a user,
**characterized in that** the at least one microbeamformer (30) includes a plurality of microbeamformer channels which are configured to operate the high frequency and low frequency transducer arrays (80H, 80L) simultaneously, wherein each channel comprises a first transmitter for driving the high frequency transducer array (80H) and a second transmitter for driving the low frequency transducer array (80L).

2. The wireless ultrasound probe of claim 1, comprising a third side comprising a third transducer array configured to operate in a different scanning mode than the high frequency and low frequency transducer arrays.

3. The wireless ultrasound probe of claim 1, wherein the probe housing has a square, rectangular, triangular, or trapezoidal shape.

4. The wireless ultrasound probe of claim 1, wherein the low frequency transducer array (80L) is arranged with respect to the probe housing such that it is enclosed by a grip of the user's hand when scanning with the high frequency transducer array (80H).

5. The wireless ultrasound probe of claim 1, further comprising at least one indicator component to identify which of the transducer arrays (80H, 80L) is selected for the ultrasound scanning procedure.

6. The wireless ultrasound probe of claim 5, wherein the at least one indicator component comprises a light.

7. The wireless ultrasound probe of claim 1, further comprising an activation component (22) on the probe housing, wherein a selection of the high frequency or low frequency transducer array (80H, 80L) defines a function of the activation component.

8. The wireless ultrasound probe of claim 7, wherein the activation component (22) is configured to be activated in line with the selected transducer array.

9. The wireless ultrasound probe of claim 1, further comprising a motion sensor configured to provide position information of the probe in relation to a patient.

10. An ultrasound diagnostic scanning system, comprising the probe of claim 1 and a display configured to wirelessly communicate with the probe.

11. The ultrasound diagnostic scanning system of claim 10, wherein the probe, the display, or both are configured to provide scanning mode presets to a user.

12. The ultrasound diagnostic scanning system of claim 11, wherein the scanning mode presets can be selected according to a user input.

13. The ultrasound diagnostic scanning system of claim 12, wherein the user input comprises a button or touch-based selection device on the probe or the display.

14. The ultrasound diagnostic scanning system of claim 12, further comprising a plurality of imaging presets comprising cardiac, abdominal, cardiac, and renal imaging presets.

15. A method for using a wireless ultrasound probe, according to claims 1-9.

## Patentansprüche

1. Eine drahtlose Ultraschallsonde (10), die Folgendes umfasst:
ein Sondengehäuse mit einer ersten Seite, einer zweiten Seite und einem Griffabschnitt, wobei die erste Seite eine Hochfrequenz-Wandleranordnung (80H) und die zweite Seite eine Niederfrequenz-Wandleranordnung (80L) umfasst, wobei die Hochfrequenz- und die Niederfrequenz-Wandleranordnungen (80H, 80L) in verschiedenen Abtastmodi ausgeführt werden;
mindestens einen Mikrobeamformer (30), der mit den Hochfrequenz- und Niederfrequenz-Wandleranordnungen (80H, 80L) verbunden ist;
einen Prozessor innerhalb des Gehäuses, der für einen Ultraschall-Abtastvorgang zwischen der Hochfrequenzoder Niederfrequenz-Wandleranordnung (80H, 80L) auswählt; und
einen Bildprozessor, der Ultraschallbilddaten zum Anzeigen für einen Benutzer erzeugt,
und die sich dadurch auszeichnet, dass der mindestens eine Mikrobeamformer (30) mehrere Mikrobeamformer-Kanäle umfasst, die die Hochfrequenz- und Niederfrequenz-Wandleranordnung (80H, 80L) gleichzeitig ausführen, wobei die einzelnen Kanäle jeweils einen ersten Sender zum Ausführen der Hochfrequenz-Wandleranordnung (80H) und einen zweiten Sender zum Ausführen der Niederfrequenz-Wandleranordnung (80L) umfassen.

2. Die drahtlose Ultraschallsonde gemäß Anspruch 1, die eine dritte Seite mit einer dritten Wandleranordnung umfasst, die in einem anderen Abtastmodus ausgeführt wird, als die Hochfrequenz- und die Niederfrequenz-Wandleranordnung.

3. Die drahtlose Ultraschallsonde gemäß Anspruch 1, wobei das Sondengehäuse eine quadratische, rechteckige, dreieckige oder trapezförmige Form aufweist.

4. Die drahtlose Ultraschallsonde gemäß Anspruch 1, wobei die Niederfrequenz-Wandleranordnung (80L) in Bezug auf das Sondengehäuse so angeordnet ist, dass sie beim Abtasten mit der Hochfrequenz-Wandleranordnung (80H) von der Hand des Benutzers umschlossen wird.

5. Die drahtlose Ultraschallsonde gemäß Anspruch 1, die zudem mindestens eine Anzeigekomponente umfasst, um ermitteln zu können, welche der Wandleranordnungen (80H, 80L) für den Ultraschall-Abtastvorgang ausgewählt wurde.

6. Die drahtlose Ultraschallsonde gemäß Anspruch 5, wobei die mindestens eine Anzeigekomponente ein Leuchte umfasst.

7. Die drahtlose Ultraschallsonde gemäß Anspruch 1, die zudem eine Aktivierungskomponente (22) am Sondengehäuse umfasst, wobei das Auswählen der Hochfrequenz- oder Niederfrequenz-Wandleranordnung (80H, 80L) eine Funktion der Aktivierungskomponente definiert.

8. Die drahtlose Ultraschallsonde gemäß Anspruch 7, wobei die Aktivierungskomponente (22) anhand der ausgewählten Wandleranordnung aktiviert wird.

9. Die drahtlose Ultraschallsonde gemäß Anspruch 1, die zudem einen Bewegungssensor umfasst, der Positionsdaten der Sonde in Bezug auf einen Patienten bereitstellt.

10. Ein Ultraschall-Diagnosesystem, das die Sonde gemäß Anspruch 1 sowie eine Anzeige umfasst und für die drahtlose Kommunikation mit der Sonde konfiguriert ist.

11. Das Ultraschall-Diagnosesystem gemäß Anspruch 10, wobei die Sonde, die Anzeige oder beide so konfiguriert sind, dass sie dem Benutzer Abtastmodus-Voreinstellungen bereitstellen.

12. Das Ultraschall-Diagnosesystem gemäß Anspruch 11, wobei die Abtastmodus-Voreinstellungen anhand einer Benutzereingabe ausgewählt werden können.

13. Das Ultraschall-Diagnosesystem gemäß Anspruch 12, wobei die Benutzereingabe an der Sonde oder Anzeige ein Tasten- oder Touch-basiertes Auswahlgerät umfasst.

14. Das Ultraschall-Diagnosesystem gemäß Anspruch 12, das zudem mehrere Voreinstellungen für die Bildgebung umfasst, darunter kardiale, abdominale, kardiale und renaleVoreinstellungen.

15. Eine Methode für das Verwenden einer drahtlosen Ultraschallsonde gemäß den Ansprüchen 1 bis 9.

## Revendications

1. Sonde à ultrasons (10) sans fil, comprenant :
un boîtier de sonde comportant un premier côté, un deuxième côté et une partie de préhension, dans laquelle le premier côté comprend un réseau de transducteurs à haute fréquence (80H) et le deuxième côté comprend un réseau de transducteurs basse fréquence (80L), dans laquelle les réseaux de transducteurs haute fréquence et basse fréquence (80H, 80L) sont conçus pour fonctionner dans différents modes de balayage ;
au moins un formateur de microfaisceaux (30) couplé aux réseaux de transducteurs haute fréquence et basse fréquence (80H, 80L) ;
un processeur dans le boîtier configuré pour sélectionner le réseau de transducteurs haute fréquence ou basse fréquence (80H, 80L) pour une procédure de balayage par ultrasons ; et
un processeur d'image configuré pour générer des données d'image par ultrasons à afficher à un utilisateur,
**caractérisé en ce que** l'au moins un formateur de microfaisceaux (30) comprend une pluralité de canaux de formateur de microfaisceaux, lesquels sont conçus pour faire fonctionner les réseaux de transducteurs haute fréquence et basse fréquence (80H, 80L) simultanément, dans laquelle chaque canal comprend un premier émetteur permettant de piloter le réseau de transducteurs haute fréquence (80H) et un second émetteur permettant de piloter le réseau de transducteurs basse fréquence (80L).

2. Sonde à ultrasons sans fil selon la revendication 1, comprenant un troisième côté comprenant un troisième réseau de transducteurs conçu pour fonctionner dans un mode de balayage différent des réseaux de transducteurs haute fréquence et basse fréquence.

3. Sonde à ultrasons sans fil selon la revendication 1, dans laquelle le boîtier de sonde présente une forme carrée, rectangulaire, triangulaire ou trapézoïdale.

4. Sonde à ultrasons sans fil selon la revendication 1, dans laquelle le réseau de transducteurs basse fréquence (80L) est agencé par rapport au boîtier de sonde de telle sorte qu'il est enfermé par une prise de la main de l'utilisateur lors du balayage à l'aide du réseau de transducteurs haute fréquence (80H).

5. Sonde à ultrasons sans fil selon la revendication 1, comprenant en outre au moins un composant indicateur permettant d'identifier lequel des réseaux de transducteurs (80H, 80L) est sélectionné pour la procédure de balayage par ultrasons.

6. Sonde à ultrasons sans fil selon la revendication 5, dans laquelle l'au moins un composant indicateur comprend une lumière.

7. Sonde à ultrasons sans fil selon la revendication 1, comprenant en outre un composant d'activation (22) sur le boîtier de sonde, dans laquelle une sélection du réseau de transducteurs haute fréquence ou basse fréquence (80H, 80L) définit une fonction du composant d'activation.

8. Sonde à ultrasons sans fil selon la revendication 7, dans laquelle le composant d'activation (22) est conçu pour être activé en ligne avec le réseau de transducteurs sélectionné.

9. Sonde à ultrasons sans fil selon la revendication 1, comprenant en outre un capteur de mouvement conçu pour fournir des informations de position de la sonde par rapport à un patient.

10. Système de balayage de diagnostic par ultrasons, comprenant la sonde selon la revendication 1 et un affichage conçu pour communiquer sans fil avec la sonde.

11. Système de balayage de diagnostic par ultrasons selon la revendication 10, dans lequel la sonde, l'affichage ou les deux sont conçus pour fournir des paramétrages antécédents de mode de balayage à un utilisateur.

12. Système de balayage de diagnostic par ultrasons selon la revendication 11, dans lequel les paramétrages antécédents de mode de balayage peuvent être sélectionnés en fonction d'une entrée utilisateur.

13. Système de balayage de diagnostic par ultrasons selon la revendication 12, dans lequel l'entrée utilisateur comprend un bouton ou un dispositif de sélection tactile sur la sonde ou l'affichage.

14. Système de balayage de diagnostic par ultrasons selon la revendication 12, comprenant en outre une pluralité de paramétrages antécédents d'imagerie comprenant des paramétrages antécédents d'imagerie cardiaque, abdominale, cardiaque et rénale.

15. Procédé d'utilisation d'une sonde à ultrasons sans fil, selon les revendications 1 à 9.
